# EUROPÄISCHE PATENTANMELDUNG

(11) **EP 1 941 918 A2**
(43) Veröffentlichungstag der Anmeldung: **09.07.2008**
(21) Anmeldenummer: 07023023.0
(22) Anmeldetag: 28.11.2007
(51) Int. Cl.: A61L 31/02, A61L 31/08, A61L 31/14

(54) **Verfahren zur Herstellung einer korrosionshemmenden Beschichtung auf einem Implantat aus einer biokorrodierbaren Magnesiumlegierung sowie nach dem Verfahren hergestelltes Implantat**

(30) Priorität: 19.12.2006 DE 102006060501
(71) Anmelder: BIOTRONIK VI Patent AG, 6341 Baar (CH)
(72) Erfinder: Kappelt, Gerhard, 91080 Uttenreuth (DE); Kurze, Peter, Prof., Dr., 52385 Nideggen (DE); Banerjee, Dora, 50171 Kerpen (DE)
(74) Vertreter: Lindner-Vogt, Karin L.

(57) **Zusammenfassung**

Die Erfindung betrifft ein Verfahren zur Herstellung einer korrosionshemmenden Beschichtung auf einem Implantat aus einer biokorrodierbaren Magnesiumlegierung sowie nach dem Verfahren erhaltene oder erhältliche Implantate. Das Verfahren umfasst die Schritte:
(i) Bereitstellen des Implantats; und
(ii) Behandeln der Implantatsoberfläche mit einer wässrigen oder alkoholischen Konversionslösung enthaltend
ein oder mehrere Ionen ausgewählt aus der Gruppe K⁺, Na⁺, NH₄⁺, Ca²⁺, Mg²⁺, Zn²⁺, Ti⁴⁺, Zr⁴⁺, Ce³⁺, Ce⁴⁺, PO₄³⁻, HPO₄²⁻, H₂PO₄⁻, OH-, BO₃³⁻, B₄O₇²⁻, SiO₃²⁻, MnO₄²⁻, MnO₄⁻, VO₃⁻, WO₄²⁻, MoO₄²⁻, TiO₃²⁻, Se²⁻, ZrO₃²⁻ und NbO₄⁻,

wobei eine Konzentration des Ions oder der Ionen jeweils im Bereich von 10⁻²mol/l bis 2 mol/l liegt.

## Beschreibung

Die Erfindung betrifft ein Verfahren zur Herstellung einer korrosionshemmenden Beschichtung auf einem Implantat aus einer biokorrodierbaren Magnesiumlegierung sowie nach dem Verfahren erhaltene oder erhältliche Implantate.

Medizinische Implantate unterschiedlichster Zweckbestimmung sind in großer Vielfalt aus dem Stand der Technik bekannt. Häufig ist nur ein zeitweiliger Verbleib des Implantats im Körper zur Erfüllung des medizinischen Zweckes erforderlich. Implantate aus permanenten Werkstoffen, also Werkstoffen, die im Körper nicht abgebaut werden, sind wieder zu entfernen, da es mittel- und langfristig auch bei hoher Biokompatibilität zu Abstoßungsreaktionen des Körpers kommen kann.

Ein Ansatz zur Vermeidung eines weiteren chirurgischen Eingriffs besteht nun darin, das Implantat ganz oder in Teilen aus einem biokorrodierbaren Werkstoff zu formen. Unter Biokorrosion werden mikrobielle Vorgänge oder schlicht durch die Anwesenheit von Körpermedien bedingte Prozesse verstanden, die zu einem allmählichen Abbau der aus dem Werkstoff bestehen Struktur führen. Zu einem bestimmten Zeitpunkt verliert das Implantat oder zumindest der Teil des Implantates, der aus dem biokorrodierbaren Werkstoff besteht, seine mechanische Integrität. Die Abbauprodukte werden vom Körper weitgehend resorbiert. Diese können, wie beispielsweise Magnesium, lokal sogar positive therapeutische Wirkung entfalten. Geringe Mengen nicht resorbierbarer Abbauprodukte sind tolerierbar.

Biokorrodierbare Werkstoffe wurden unter anderem auf Basis von Polymeren synthetischer Natur oder natürlichen Ursprungs entwickelt. Die mechanischen Materialeigenschaften (geringe Plastizität), aber auch teils die geringe Biokompatibilität der Abbauprodukte der Polymere (teils erhöhte Thrombogenität, vermehrte Inflammation), limitieren den Einsatz jedoch deutlich. So müssen beispielsweise orthopädische Implantate häufig hohen mechanischen Beanspruchungen standhalten und vaskuläre Implantate, z. B. Stents, je nach Design sehr speziellen Anforderungen an E-Modul, Brüchigkeit und Formbarkeit genügen.

Ein aussichtsreicher Ansatz zur Lösung des Problems liegt in der Verwendung biokorrodierbarer Metalllegierungen. So wird in DE 197 31 021 A1 vorgeschlagen, medizinische Implantate aus einem metallischen Werkstoff zu formen, dessen Hauptbestandteil ein Element aus der Gruppe Alkalimetalle, Erdalkalimetalle, Eisen, Zink und Aluminium ist. Als besonders geeignet werden Legierungen auf Basis von Magnesium, Eisen und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium > 90%, Yttrium 3,7 - 5,5%, Seltenerdmetallen 1,5 - 4,4% und Rest < 1 % bekannt, die sich insbesondere zur Herstellung einer Endoprothese, z. B. in Form eines selbstexpandierenden oder ballonexpandierbaren Stent, eignet. Ungeachtet der erreichten Fortschritte auf dem Gebiet biokorrodierbarer Metalllegierungen sind die bisher bekannten Legierungen aufgrund ihres Korrosionsverhaltens nur beschränkt einsatzfähig. Die relativ rasche Biokorrosion der Magnesiumlegierungen, insbesondere im Bereich mechanisch stark belasteter Strukturen, limitiert den Einsatz.

Sowohl die Grundlagen der Magnesium-Korrosion als auch eine große Anzahl von technischen Verfahren zur Verbesserung des Korrosionsverhaltens (im Sinne einer Verstärkung des Korrosionsschutzes) sind aus dem Stand der Technik bekannt. Bekannt ist beispielsweise, dass der Zusatz von Yttrium und/oder weiteren Seltenerdmetallen einer Magnesiumlegierung einen leicht erhöhten Korrosionswiderstand in Meerwasser verleiht.

Ein Ansatzpunkt sieht vor, eine korrosionsschützende Schicht auf dem aus Magnesium oder einer Magnesiumlegierung bestehenden Formkörper zu erzeugen. Bekannte Verfahren zur Erzeugung einer korrosionsschützenden Schicht wurden unter dem Gesichtpunkt eines technischen Einsatzes des Formkörpers - jedoch nicht medizin-technischen Einsatzes in biokorrodierbaren Implantaten in physiologischer Umgebung - entwickelt und optimiert. Diese bekannten Verfahren umfassen: das Aufbringen von Polymeren oder anorganischen Deckschichten, das Erzeugen einer Emaille, die chemische Konversion der Oberfläche, Heißgasoxidation, Anodisieren, Plasmaspritzen, Laserstrahl-Umschmelzen, PVD-Verfahren, Ionenimplantation oder Lackieren.

Herkömmliche technische Einsatzgebiete von Formkörpern aus Magnesiumlegierungen außerhalb der Medizintechnik erfordern in der Regel eine weitgehende Unterbindung korrosiver Prozesse. Dementsprechend ist die Zielstellung der meisten technischen Verfahren eine vollständige Inhibierung korrosiver Prozesse. Dagegen sollte die Zielstellung zur Verbesserung des Korrosionsverhaltens von biokorrodierbaren Magnesiumlegierungen nicht in der vollständigen Unterbindung, sondern nur in der Hemmung korrosiver Prozesse liegen. Schon aus diesem Grunde eignen sich die meisten bekannten Verfahren zur Erzeugung einer Korrosionsschutzschicht nicht. Ferner müssen für einen medizin-technischen Einsatz auch toxikologische Aspekte berücksichtigt werden. Des Weiteren sind korrosive Prozesse stark von dem Medium abhängig, in dem sie ablaufen, und daher dürfte eine Übertragbarkeit der unter herkömmlichen Umweltbedingungen auf technischem Gebiet gewonnenen Erkenntnisse zum Korrosionsschutz auf die Prozesse in physiologischer Umgebung nicht uneingeschränkt möglich sein. Schließlich dürften bei einer Vielzahl von medizinischen Implantaten auch die der Korrosion zugrunde liegenden Mechanismen von üblichen technischen Anwendungen des Werkstoffs abweichen. So werden beispielsweise Stents, chirurgisches Nahtmaterial oder Clips im Gebrauch mechanisch verformt, so dass der Teilprozess der Spannungsriss-Korrosion beim Abbau dieser Formkörper erhebliche Bedeutung haben dürfte.

DE 101 63 106 A1 sieht vor, den Magnesiumwerkstoff durch Modifikation mit Halogeniden in seiner Korrosivität zu verändern. Der Magnesiumwerkstoff soll zur Herstellung medizinischer Implantate verwendet werden. Bevorzugt ist das Halogenid ein Fluorid. Die Modifikation des Werkstoffs wird durch Zulegieren salzförmiger Halogenverbindungen erreicht. Es wird demnach die Zusammensetzung der Magnesiumlegierung durch Zugabe der Halogenide verändert, um die Korrosionsrate zu mindern. Dementsprechend wird der gesamte, aus einer solchen modifizierten Legierung bestehende Formkörper ein verändertes Korrosionsverhalten besitzen. Durch das Zulegieren können jedoch weitere Werkstoffeigenschaften beeinflusst werden, die bei der Verarbeitung von Bedeutung sind oder auch die mechanischen Eigenschaften des aus dem Werkstoff entstehenden Formkörpers prägen.

Der vorliegenden Erfindung liegt die Aufgabe zugrunde, ein zumindest alternatives oder vorzugsweise verbessertes Verfahren zur Herstellung einer korrosionshemmenden Beschichtung auf einem Implantat aus einer biokorrodierbaren Magnesiumlegierung bereitzustellen. Die durch das Verfahren erschaffene korrosionshemmende Beschichtung soll nur eine temporäre Inhibition, aber nicht vollständige Unterbindung der Korrosion des Werkstoffs in physiologischer Umgebung bewirken

Diese Aufgabe wird nach einem ersten Aspekt der Erfindung durch das erfindungsgemäße Verfahren zur Herstellung einer korrosionshemmenden Beschichtung auf einem Implantat aus einer biokorrodierbaren Magnesiumlegierung gelöst. Dazu umfasst das Verfahren die Schritte:
(i) Bereitstellen des Implantats; und
(ii) Behandeln der Implantatoberfläche mit einer wässrigen oder alkoholischen Konversionslösung enthaltend
   ein oder mehrere Ionen ausgewählt aus der Gruppe K⁺, Na⁺, NH₄⁺, Ca²⁺, Mg²⁺, Zn²⁺ , Ti⁴⁺, Zr⁴⁺, Ce³⁺, Ce⁴⁺, PO₄³⁻ , HPO₄²⁻, H₂PO₄⁻, OH⁻, BO₃³⁻, B₄O₇²⁻, SiO₃²⁻, MnO₄²⁻, MnO₄⁻, VO₃⁻, WO₄²⁻, MoO₄²⁻, TiO₃²⁻, Se²⁻, ZrO₃²- und NbO₄⁻,
   wobei eine Konzentration des Ions oder der Ionen jeweils im Bereich von 10⁻² mol/l bis 2 mol/l liegt.

Es hat sich gezeigt, dass die Erzeugung einer Beschichtung in der genannten Verfahrensweise nicht zur Ausbildung einer vollständig oder weitgehend die Korrosion in physiologischer Umgebung inhibierenden Schutzschicht führt. Mit anderen Worten, in physiologischer Umgebung erfolgt dennoch eine Korrosion des Implantats, jedoch mit deutlich verzögerter Geschwindigkeit. Die Behandlung der Implantatoberfläche mit der Konversionslösung bedingt eine anodische Oxidation des Implantats. Sie wird entweder ohne Verwendung einer äußeren Stromquelle (außen stromlos) oder mit einer Stromquelle durchgeführt.

Die korrosionshemmende Beschichtung entsteht demnach durch oberflächennahe Konversion des Werkstoffs des Implantats; es erfolgt also kein Auftrag von Material auf eine Oberfläche des Implantats, sondern es findet eine chemische Umwandlung (Konversion) der metallischen Oberfläche und der verschiedenen Bestandteile der Konversionslösung statt.

Von den aufgeführten Ionen erfüllen OH⁻-Ionen in einem wässrigen oder alkoholischen System eine besondere Funktion. Sie bilden auf der Oberfläche des Implantats eine stabile Barriereschicht aus Mg(OH)₂ unterhalb eines von den weiteren Ionen gebildeten Teils der Konversionsschicht. Die Barriereschicht behindert die Diffusion von korrosionsfördernden Ionen in das Metall und ist hochgradig duktil bei mechanischen Verformungen. Vorzugsweise enthält die Konversionslösung daher OH⁻-Ionen und ein oder mehrere lonen ausgewählt aus der Gruppe K⁺, Na⁺, NH₄⁺, Ca²⁺, Mg²⁺, Zn²⁺, Ti⁴⁺, Zr⁴⁺, Ce³⁺, Ce⁴⁺, PO₄³⁻, HPO₄²⁻, H₂PO₄⁻, OH, BO₃³⁻, B₄O₇²⁻, SiO₃²⁻, MnO₄²⁻, MnO₄⁻, VO₃⁻, WO₄²⁻, MoO₄²⁻, TiO₃²⁻, Se²⁻, ZrO₃²⁻ und NbO₄⁻.

Auf der vorgenannten Barriereschicht bilden sich insbesondere aus wässrigen oder alkoholischen Konversionslösungen mit den Anionen PO₄³⁻, H₂PO₄⁻, HPO₄²⁻, BO₃³⁻, B₄O₇²⁻ und SiO₃²⁻ Deckschichten mit geringer Löslichkeit aus und schützen dadurch das Implantat zusätzlich. Außerdem sind diese Deckschichten ebenfalls duktil, so dass sie bei mechanischer Verformung des Implantats nicht abplatzen. Vorzugsweise enthält die Konversionslösung daher OH-Ionen und ein oder mehrere Anionen ausgewählt aus der Gruppe PO₄³⁻, H₂PO₄⁻, HPO₄²⁻, BO₃³⁻, B₄O₇²⁻ und SiO₃²⁻.

Von den genannten Kationen sind K⁺, Na⁺, NH₄⁺, Ca²⁺ und Mg²⁺ bereits im Körper vorhanden, so dass nach Möglichkeit lösliche Salze derselben mit den vorhandenen Anionen Einsatz finden, wie zum Beispiel NaH₂PO₄, Na₂B₄O₇ oder Mg(MnO₄)₂. Vorzugsweise enthält die Konversionslösung daher ein oder mehrere Kationen ausgewählt aus der Gruppe K⁺, Na⁺, NH₄⁺, Ca²⁺ und Mg²⁺.

Schließlich dienen die Ionen MnO₄²⁻, MnO₄⁻, VO₃⁻, WO₄ ²⁻, MoO₄²⁻, TiO₃²⁻, ZrO₃²⁻ und NbO₄⁻ im Redoxsystem als Oxidationsmittel, die den elektrochemischen Vorgang einleiten und aufrecht erhalten, der zur Ausbildung der Konversionsschicht führt. Vorzugsweise enthält die Konversionslösung daher ein oder mehrere Anionen ausgewählt aus der Gruppe MnO₄²⁻, MnO₄⁻, VO₃⁻, WO₄²⁻, MoO₄²⁻, TiO₃²⁻, ZrO₃²⁻ und NbO₄⁻.

Eine besonders bevorzugte Konversionslösung enthält:
(i) OH⁻;
(ii) ein oder mehrere Anionen ausgewählt aus der Gruppe PO₄³⁻ , H₂PO₄⁻, HPO₄²⁻, BO₃³⁻, B₄O₇²⁻ und SiO₃²⁻ zur Bildung einer Deckschicht;
(iii) ein oder mehrere Kationen ausgewählt aus der Gruppe K⁺, Na⁺, NH₄⁺, Ca²⁺ und Mg²⁺; und
(iv) ein oder mehrere Anionen ausgewählt aus der Gruppe MnO₄²⁻, MnO₄⁻, VO₃⁻, WO₄²⁻, MoO₄²⁻, TiO₃²⁻, ZrO₃²⁻ und NbO₄⁻ als Oxidationsmittel.

Optional enthält die Konversionslösung Puffer, insbesondere alkalische Puffer wie EDTA, Ethylendiamin, Hexamethylentetramin. Alkalische Puffer unterstützen die Ausbildung der Barriereschicht durch ihr hohes Angebot an OH⁻-Ionen. Weiterhin wirken sie sich günstig auf die Stabilität der Konversionslösung aus.

Das Implantat besteht ganz oder in zumindest zu Teilen aus der biokorrodierbare Magnesiumlegierung. Unter Legierung wird vorliegend ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%.

Die Magnesiumlegierung enthält vorzugsweise Yttrium und weitere Seltenerdmetalle, da sich eine derartige Legierung aufgrund ihrer physiko-chemischen Eigenschaften und hohen Biokompatibilität, insbesondere auch seiner Abbauprodukte, auszeichnet.

Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. sie zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

Die Magnesiumlegierung ist so in ihrer Zusammensetzung zu wählen, dass sie biokorrodierbar ist. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Der Begriff Korrosion bezieht sich vorliegend auf die Reaktion eines metallischen Werkstoffes mit seiner Umgebung, wobei eine messbare Veränderung des Werkstoffs bewirkt wird, die - bei Einsatz des Werkstoffs in einem Bauteil - zu einer Beeinträchtigung der Funktion des Bauteils führt. Ein Korrosionssystem besteht vorliegend aus dem korrodierenden metallischen Werkstoff sowie einem flüssigen Korrosionsmedium, das in seiner Zusammensetzung die Verhältnisse in physiologischer Umgebung nachstellt oder ein physiologisches Medium, insbesondere Blut ist. Werkstoffseitig beeinflussen die Korrosion Faktoren, wie die Zusammensetzung und Vorbehandlung der Legierung, mikro- und submikroskopische Inhomogenitäten, Randzoneneigenschaften, Temperatur- und mechanischer Spannungszustand und insbesondere die Zusammensetzung einer die Oberfläche bedeckenden Schicht. Auf Seiten des Mediums wird der Korrosionsprozess durch Leitfähigkeit, Temperatur, Temperaturgradienten, Azidität, Volumen-Oberflächen-Verhältnis, Konzentrationsunterschied sowie Strömungsgeschwindigkeit beeinflusst.

An der Phasengrenzfläche zwischen Werkstoff und Medium laufen Redox-Reaktionen ab. Für eine schützende bzw. hemmende Wirkung müssen vorhandene Schutzschichten und/oder die Produkte der Redox-Reaktionen eine gegen das Korrosionsmedium ausreichend dichte Struktur ausbilden, eine bezogen auf die Umgebung erhöhte thermodynamische Stabilität aufweisen und im Korrosionsmedium wenig löslich oder unlöslich sein. In der Phasengrenzfläche, genauer in einer sich in diesem Bereich ausbildenden Doppelschicht, laufen Ad- und Desorptionsprozesse ab. Die Vorgänge in der Doppelschicht sind geprägt von den dort ablaufenden kathodischen, anodischen und chemischen Teilprozessen. Bei Magnesiumlegierungen ist in der Regel eine allmähliche Alkalisierung der Doppelschicht zu beobachten. Fremdstoffablagerungen, Verunreinigungen und Korrosionsprodukte beeinflussen den Korrosionsprozess. Die Vorgänge bei der Korrosion sind demnach hoch komplex und lassen sich gerade im Zusammenhang mit einem physiologischen Korrosionsmedium, also Blut oder künstlichem Plasma, nicht oder nur im geringen Umfang voraussagen, da Vergleichsdaten fehlen. Schon aus diesem Grunde ist das Auffinden einer korrosionshemmenden Beschichtung, d. h. einer Beschichtung, die nur zur temporären Herabsetzung der Korrosionsrate eines metallischen Werkstoffs der weiter oben genannten Zusammensetzung in physiologischer Umgebung dient, eine außerhalb der Routine eines Fachmanns liegende Maßnahme. Dies gilt in besonderem Maße für Stents, welche zum Zeitpunkt der Implantation lokal hohen plastischen Verformungen ausgesetzt sind. Konventionelle Ansätze mit starren korrosionshemmenden Schichten sind für derartige Voraussetzungen ungeeignet.

Der Vorgang der Korrosion lässt sich durch Angabe einer Korrosionsrate quantifizieren. Ein zügiger Abbau ist mit einer hohen Korrosionsrate verbunden, und umgekehrt. Bezogen auf den Abbau des gesamten Formkörpers wird eine im Sinne der Erfindung modifizierte Oberfläche zur Herabsetzung der Korrosionsrate führen. Die erfindungsgemäße korrosionshemmende Beschichtung kann im Laufe der Zeit selbst abgebaut werden bzw. kann die von ihr abgedeckten Bereiche des Implantats nur noch in einem immer geringeren Umfang schützen. Daher ist der Verlauf der Korrosionsrate für das gesamte Implantat nicht linear. Vielmehr ergibt sich zu Beginn der einsetzenden korrosiven Prozesse eine relativ niedrige Korrosionsrate, die im Laufe der Zeit ansteigt. Dieses Verhalten wird als temporäre Herabsetzung der Korrosionsrate im Sinne der Erfindung verstanden und zeichnet die korrosionshemmende Beschichtung aus. Im Fall von Koronarstents sollte die mechanische Integrität der Struktur über ein Zeitraum von drei Monaten nach Implantation aufrechterhalten werden.

Vorzugsweise erfolgt die Behandlung im Schritt (ii) durch anodische Oxidation unter Anlegen einer Spannung an das Implantat. Dabei wird das zu behandelnde Implantat in eine elektrisch leitende Flüssigkeit gebracht (Elektrolyt), wo es als Anode an eine Gleichspannungsquelle angeschlossen wird. Die Kathode besteht meist aus nicht rostendem Stahl, Blei oder Aluminium. Im so entstandenen Spannungsfeld wandern Anionen zur Implantatoberfläche. Dort reagieren sie mit dem Werkstoff, und es bildet sich eine Konversionsschicht. Bei wässrigen Medien kann sich an der Kathode Wasserstoff bilden, der in Form von Gas entweicht. Die entstandene Beschichtung kann auch einen mehrschichtigen Aufbau aufweisen, z. B. kann eine dünne Sperrschicht, die fast porenfrei, äußerst dicht und elektrisch isolierend ist, und eine weit voluminösere, leicht poröse Deckschicht, die sich durch eine chemische Reaktion der Sperrschicht mit dem Elektrolyten bildet, vorhanden sein.

Als Elektrolyte für die anodische Oxidation mit äußerer Stromquelle werden vorzugsweise Konversionslösungen eingesetzt, die ein oder mehrere Ionen ausgewählt aus der Gruppe NH₄⁺, PO₄³⁻ und/oder BO₃³⁻ enthalten.

Die anodische Oxidation kann auch mit äußerer Stromquelle unter Plasmaentladung erfolgen. Der Magnesiumstent wird dabei elektrisch kontaktiert und mit einer hohen Spannung von über 100 Volt beaufschlagt. Dadurch entsteht ein Plasma (Funken) auf der Oberfläche des Stents, wodurch die Materialoberfläche zu einer Oxidkeramikschicht umgewandelt wird.

Alternativ zur anodischen Oxidation mit äußerer Stromquelle kann die Behandlung im Schritt (ii) auch ohne äußere Stromquelle erfolgen. Durch Redox-Reaktionen an der Oberfläche des Werkstoffs entsteht die korrosionshemmende Beschichtung. Vorzugsweise enthält die Konversionslösung dabei ein oder mehrere Ionen ausgewählt aus der Gruppe K⁺, Na⁺ NH₄⁺, MnO₄ und VO₃⁻.

Diese Redoxreaktion wird durch Kontaktierung des Magnesiumwerkstoffes mit einem Edelmetall im Elektrolyten verstärkt. Durch die unterschiedlichen elektrochemischen Potenziale der Magnesiumlegierung und des Edelmetalls stellt sich eine höhere Potenzialdifferenz ein.

Bei der Kombination Magnesium (Potenzial - 2,37 Volt) beispielsweise mit reinem Platin (Potenzial + 1,60 Volt) beträgt die Differenz 3,97 Volt. Diese Spannung reicht aus, um die Redoxreaktion zu initiieren und eine Konversionsschicht in dem betreffenden Elektrolyten zu erzeugen.

In einer optionalen Ausführung wird zusätzlich in Schritt (i) der Behandlung das Implantat mit einem Edelmetall kontaktiert. Bevorzugt umfassen diese Edelmetalle PT, Au, Rh und Ru.

Ein zweiter Aspekt der Erfindung bezieht sich auf ein Implantat, das nach dem vorab beschriebenen Verfahren hergestellt wird.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren. Das Implantat besteht ganz oder in Teilen aus dem biokorrodierbaren Werkstoff. Wenn das Implantat nur in Teilen aus dem biokorrodierbaren Werkstoff besteht, so ist dieser Teil entsprechend zu beschichten.

Vorzugsweise ist das Implantat ein Stent. Stents herkömmlicher Bauart weisen eine filigrane Struktur aus metallischen Streben auf, die zur Einbringung in den Körper zunächst in einem nicht-expandierten Zustand vorliegt und die am Ort der Applikation dann in einen expandierten Zustand aufgeweitet wird. Bei Stents bestehen besondere Anforderungen an die korrosionshemmende Schicht: Die mechanische Belastung des Materials während der Expansion des Implantats (Dilatation) hat Einfluss auf den Verlauf des Korrosionsprozesses und es ist davon auszugehen, dass die Spannungsrisskorrosion in den belasteten Bereichen verstärkt wird. Eine korrosionshemmende Schicht sollte diesen Umstand berücksichtigen. Weiterhin könnte eine harte korrosionshemmende Schicht während der Expansion des Stents abplatzen und eine Rissbildung in der Schicht bei Expansion des Implantats dürfte unvermeidbar sein. Schließlich sind die Dimensionen der filigranen metallischen Struktur zu beachten und es sollte nach Möglichkeit nur eine dünne, aber auch gleichmäßige korrosionshemmende Schicht erzeugt werden. Es hat sich nun gezeigt, dass das Aufbringen der erfindungsgemäßen Beschichtung ganz oder zumindest weitgehend diesen Anforderungen genügt.

Vorzugsweise weist die durch Behandlung mit der Konversionslösung erhältliche korrosionshemmende Beschichtung eine Schichtdicke in Bereich von 300 nm bis 20 µm, insbesondere im Bereich von 800 nm bis 10 µm auf.

Die Erfindung wird nachfolgend anhand von Ausführungsbeispielen näher erläutert.

### Ausführungsbeispiel 1 - Anodische Oxidation ohne äußere Stromquelle (Tauchverfahren)

Stents aus der biokorrodierbaren Magnesiumlegierung WE43 (93 Gew.% Magnesium, 4 Gew.% Yttrium (W) und 3 Gew.% Seltenerdmetalle (E) außer Yttrium) wurden mit Isopropanol unter Ultraschall gewaschen und anschließend für 30 Sekunden in 10%iger Flusssäure gebeizt. Nach mehrfachen Spülen mit deionisiertem Wasser wurde der Stent in nassem Zustand für 5 Minuten in eine auf 30°C erwärmte, wässrige Konversionslösung der Zusammensetzung 3g/l KMnO₄ und 1 g/l NH₄VO₃ getaucht. Der pH-Wert der Konversionslösung beträgt 7,5 +/- 0,2. Nach dem Herausnehmen des Stents aus der Konversionslösung wurde das Implantat mit seiner braunen Konversionsschicht mehrfach mit deionisiertem Wasser gespült und danach für 30 Minuten im Umlufttrockner bei 120°C getrocknet.

Es wurden folgende Versuche zur Charakterisierung des Degradationsverhaltens durchgeführt:
(i) Die Stents wurden bei Raumtemperatur für 4h in künstliches Plasma gelegt, wieder entnommen und optisch nach hinsichtlich des Zustands der Degradation beurteilt.
(ii) Die Stents wurden bei Raumtemperatur für 4h in künstliches Plasma ausgelagert. Gleichzeitig wurde periodisch ein Polarisationswiderstand erfasst.
(iii) Die Stents wurden bei Raumtemperatur für 4h in künstliches Plasma ausgelagert. Aus der Lösung wurde periodisch die Elutionsrate signifikanter aus der Legierung gelöster Ionen ermittelt.
(iv) Die Stent wurden im Tier implantiert. Es folgte eine histologische Beurteilung, µ-CT-Analyse und Analyse der Zusammensetzung der in-vivo degradierten Explantate.

### Ausführungsbeispiel 2 - Anodische Oxidation ohne äußere Stromquelle (Tauchverfahren)

Stents aus der Magnesiumlegierung WE 43 wurden mit Isopropanol unter Ultraschall gewaschen und anschließend kurz mit demineralisiertem Wasser benetzt.
Der nasse Stent wurde in die Konversionslösung getaucht.

Die Konversionslösung hat folgende Zusammensetzung:

| | |
|---|---|
| NaMnO₄ | : 2,7 g/l |
| NH₄VO₃ | : 1,0 g/l |
| pH | : 7,5 ± 0,2 |

Parameter für die anodische Oxidation ohne äußere Stromquelle:

| | |
|---|---|
| Badtemperatur | : 30°C |
| Behandlungsdauer | : 10 min |

Nach dem Entfernen des Stents aus der Konversionslösung wurde dieser mehrfach gespült und anschließend für 30 Minuten bei 120°C getrocknet.

### Ausführungsbeispiel 3 - Anodische Oxidation mit äußerer Stromquelle

Stents aus der biokorrodierbaren Magnesiumlegierung WE 43 wurden mit Isopropanol unter Ultraschall gewaschen und anschließend kurz mit demineralisiertem Wasser benetzt. Der nasse Stent wurde als Anode gepolt und in einen Konversionselektrolyten eingebracht.
Der Elektrolyt hat folgende Zusammensetzung:

| | |
|---|---|
| NaMnO₄ | : 2,7 g/l |
| NH₄VO₃ | : 1,0 g/l |
| pH | : 7,5 ± 0,2 |

Die Parameter der anodischen Oxidation mit äußerer Stromquelle waren:

| | |
|---|---|
| Spannung | : 5 V |
| Badtemperatur | : 30°C |
| Behandlungsdauer | : 5 min |
| Strom | : 20 mA |

Nach der Anodisation wurde der Stent in deminiralisiertem Wasser gut gespült und für 30 Minuten bei 120°C getrocknet.
Die erhaltene Konversionsschicht war 2 bis 3 µm dick.

### Ausführungsbeispiel 4 - Anodische Oxidation in Kontakt mit Edelmetallen

Stents aus der biokorrodierbaren Magnesiumlegierung WE 43 wurden vorbehandelt wie in Ausführungsbeispiel 3; der Konversionselektrolyt hatte dieselbe Zusammensetzung wie in Ausführungsbeispiel 3.
Der Stent wurde fest an einem Platinblech kontaktiert und in die Konversionlösung getaucht.

Parameter für die anodische Oxidation in Kontakt mit Edelmetallen:

| | |
|---|---|
| Badtemperatur | : 30°C |
| Behandlungsdauer | : 10 min |

Die Nachbehandlung des beschichteten Stents erfolgte in derselben Weise wie in Ausführungsbeispiel 3.
Die erhaltene Konversionsschicht war ca. 2 µm dick.

### Ausführungsbeispiel 5 - Anodische Oxidation mit äußerer Stromquelle unter Plasmaentladung im Elektrolyten

Stents aus der biokorrodierbaren Magnesiumlegierung WE 43 wurden vor behandelt wie in Ausführungsbeispiel 3.
Der nasse Stent wurde als Anode gepolt und in einen wässrigen Konversionselektrolyten folgender Zusammensetzung eingebracht:

| |
|---|
| 0,52 mol/l PO₄³⁻ |
| 0,57 mol/l BO₃³⁻ |
| 0,40 mol/l NH4+ |
| 2,5 mol/l Hexamethylentetramin |

Der Elektrolyt hatte einen pH-Wert von 7,2.

Die Parameter der anodischen Oxidation unter Plasmaentladung waren:

| | |
|---|---|
| Stromdichte | : 1 - 1,4 A/ dm² |
| Badtemperatur | : 37 - 40°C |
| Spannung | : begrenzt auf 340 V |

Nach 5 Minuten Expositionszeit hatte die Schicht auf dem Stent eine Dicke von ca. 5 µm.
Der Stent wurde mit demineralisiertem Wasser gespült und getrocknet.

## Patentansprüche

1. Verfahren zur Herstellung einer korrosionshemmenden Beschichtung auf einem Implantat aus einer biokorrodierbaren Magnesiumlegierung, umfassend die Schritte:
(i) Bereitstellen des Implantats; und
(ii) Behandeln der Implantatoberfläche mit einer wässrigen oder alkoholischen Konversionslösung enthaltend
ein oder mehrere Ionen ausgewählt aus der Gruppe K⁺, Na⁺, NH₄⁺, Ca²⁺, Mg²⁺, Zn²⁺, Ti⁴⁺, Zr⁴⁺, Ce³⁺, C⁴⁺, PO₄³⁻, HPO₄²⁻, H₂PO₄⁻, OH⁻, BO₃³⁻, B₄O₇²⁻ , SiO₃²⁻, MnO₄²⁻, MnO₄⁻, VO₃⁻, WO₄²⁻, MoO₄²⁻, TiO₃²⁻, Se²⁻, ZrO₃²⁻ und NbO₄⁻,
wobei eine Konzentration des Ions oder der Ionen jeweils im Bereich von 10⁻² mol/l bis 2 mol/l liegt.

2. Verfahren nach Anspruch 1, bei dem die Konversionslösung OH⁻-Ionen und ein oder mehrere Ionen ausgewählt aus der Gruppe K⁺, Na⁺, NH₄⁺, Ca²⁺, Mg²⁺, Zn²⁺, Ti⁴⁺, Zr⁴⁺, Ce³⁺, Ce⁴⁺, PO₄³⁻, HPO₄²⁻, H₂PO₄⁻, OH⁻, BO₃³⁻, B₄O₇²⁻, SiO₃²⁻, MnO₄²⁻, MnO₄⁻, VO₃⁻, WO₄²⁻, MoO₄²⁻, TiO₃²⁻, Se²⁻, ZrO₃²⁻ und NbO₄⁻ enthält.

3. Verfahren nach Anspruch 1 oder 2, bei dem die Konversionslösung ein oder mehrere Kationen ausgewählt aus der Gruppe K⁺, Na⁺, NH₄⁺, Ca²⁺ und Mg²⁺ enthält.

4. Verfahren nach einem der vorhergehenden Ansprüche, bei dem die Konversionslösung ein oder mehrere Anionen ausgewählt aus der Gruppe MnO₄²⁻, MnO₄⁻, VO₃⁻, WO₄²⁻, MoO₄²⁻, TiO₃²⁻, ZrO₃²⁻ und NbO₄⁻ enthält.

5. Verfahren nach Anspruch 1, bei dem die Konversionslösung
(i) OH⁻;
(ii) ein oder mehrere Anionen ausgewählt aus der Gruppe PO₄³⁻, H₂PO₄⁻, HPO₄²⁻, BO₃³⁻, B₄O₇²⁻ und SiO₃²⁻ zur Bildung einer Deckschicht;
(iii) ein oder mehrere Kationen ausgewählt aus der Gruppe K⁺, Na⁺, NH₄⁺, Ca²⁺ und Mg²⁺; und
(iv) ein oder mehrere Anionen ausgewählt aus der Gruppe MnO₄²⁻, MnO₄⁻, VO₃⁻, WO₄²⁻, MoO₄²⁻, TiO₃²⁻, ZrO₃²⁻ und NbO₄⁻ als Oxidationsmittel
enthält.

6. Verfahren nach einem der vorhergehenden Ansprüche , bei dem die Behandlung im Schritt (ii) durch anodische Oxidation unter Anlegen einer Spannung an das Implantat erfolgt.

7. Verfahren nach Anspruch 6, bei dem die zur anodischen Oxidation eingesetzte Konversionslösung ein oder mehrere Ionen ausgewählt aus der Gruppe NH₄⁺, PO₄³⁻ und BO₃³⁻ enthält.

8. Verfahren nach Anspruch 7, bei dem die anodische Oxidation mit äußerer Stromquelle unter Plasmaentladung erfolgt.

9. Verfahren nach Anspruch 1, bei dem die Behandlung im Schritt (ii) durch Eintauchen des Implantats in die Konversionslösung erfolgt.

10. Verfahren nach Anspruch 8, bei dem die Konversionslösung ein oder mehrere Ionen ausgewählt aus der Gruppe K⁺, Na⁺, NH₄⁺, MnO₄³- und VO₃⁻ enthält.

11. Verfahren nach Anspruch 1, bei dem die Behandlung in Schritt (i) das Kontaktieren des Implantats mit einem Edelmetall umfasst.

12. Verfahren nach Anspruch 10, bei dem das Edelmetall aus der Gruppe Pt, Au, Rh, Ru verwendet wird.

13. Implantat erhalten oder erhältlich nach einem Verfahren gemäß einem der Ansprüche 1 bis 12.

14. Implantat nach Anspruch 13, bei dem das Implantat ein Stent ist.

15. Implantat nach Anspruch 13 oder 14, bei dem das Implantat eine durch die Behandlung mit der Konversionslösung erhältliche korrosionshemmende Beschichtung mit einer Schichtdicke in Bereich von 300 nm bis 20 µm aufweist.
